Europäisches Patentamt

(19) European Patent Office

Office européen des brevets

(11) Publication number: **0 001 718**
**B1**

(12) **EUROPEAN PATENT SPECIFICATION**

(45) Date of publication of patent specification: **20.01.82**

(21) Application number: **78300537.4**

(22) Date of filing: **24.10.78**

(51) Int. Cl.³: **A 61 M 1/00,**
**A 61 M 25/00,**
**A 61 B 17/22, A 61 B 10/00**

(54) **Apparatus and method for use in the removal of material from body cavities by suction.**

(30) Priority: **24.10.77 HU DO000416**

(43) Date of publication of application:
**02.05.79 Bulletin 79/9**

(45) Publication of the grant of the European patent:
**20.01.82 Bulletin 82/3**

(84) Designated Contracting States:
**BE CH DE FR GB NL SE**

(56) References cited:
**DD - A - 116 393**
**DE - A - 2 428 714**
**DE - A - 2 552 378**
**DE - U - 6 903 004**
**DE - U - 7 203 913**
**GB - A - 1 273 387**
**GB - A - 1 405 556**
**US - A - 2 715 899**
**US - A - 3 774 612**
**US - A - 3 833 000**
**US - A - 3 877 464**
**US - A - 3 929 133**
**US - A - 4 050 466**

(73) Proprietor: **CHINOIN Gyogyszer és Vegyészeti**
**Termékek Gyára RT**
**To u.1-5**
**1045 Budapest, IV (HU)**

(72) Inventor: **Balazs, Dolhay**
**Spahi u. 8.**
**Budapest, II (HU)**
Inventor: **Zsolt, Barczy**
**Martirok u.64**
**Budapest, II (HU)**

(74) Representative: **Boyes, Kenneth Aubrey et al,**
**Frank B. Dehn & Co. Imperial House 15-19**
**Kingsway**
**London WC2B 6UZ (GB)**

Apparatus and method for use in the removal of material from body cavities by suction

This invention relates to the removal of material from body cavities by suction. Such removal is effected by means of a vacuum suction unit, a catheter, and a container disposed therebetween, the container including a filter.

Removal of material from body cavities is well known in medical practice, and a number of apparatuses have been proposed. United Kingdom Patent 1,273,387 for example discloses an instrument intended mainly for gynaecological purposes, and including a catheter connected to a container provided with a filter which container serves also as a handle for the instrument.

A problem with such apparatus is that the material drawn from the cavity cannot be directly observed. Due to the fast rate of coagulation, tissue portions and clotted blood or other unnecessary material cannot be efficiently separated, in spite of the provision of a filter. Furthermore, no provision is made for the fixing and disinfection of tissue portions, preferably with formalin, which is necessary for subsequent, e.g. microscopic, examination.

In U.S. Patent 3,929,133 there is proposed apparatus for use in the removal of material from body cavities by suction, comprising a catheter, a suction unit, and a collection container disposed between the catheter and the suction unit, said container being provided with a filter in the flow path between the catheter and suction unit and being connectable to a source of liquid for washing the material collected in the container.

With such an arrangement, besides filtering of biopsy material obtained from the cavity there is possible its examination by eye, and its washing for immediate examination. This is of use, especially in gynaecology where careful inspection of the removed endometrium may influence decisively the further course of an operation. The immediate washing of withdrawn material is desirable for such inspection. Direction of the further examination of the removed material could be decided in the course of such visual evaluation. For this purpose the continuous and immediate removal of material such as blood which would interfere with the evaluation, is advantageous.

It is desirable not only to wash the material, but to treat it, e.g. to preserve the structure of the material to be examined, as soon as possible. Removal of material from a cavity generally involves substantial bleeding. Thus a major part of the extracted material is blood, disturbing examination of the cells and tissue portions, or even making it impossible. With known apparatus and techniques it has been the case that a significant part of the extracted material is not available for detailed examination. Whilst an arrangement of U.S. Patent 3,929,133 permits visual examination, any more detailed examination would involve removal of the material from the container.

According to the invention therefore, there is provided apparatus for use in the removal of material from body cavities by suction, comprising a catheter, a suction unit, and a collection container disposed between the catheter and the suction unit, said container being provided with a filter in the flow path between the catheter and suction unit and being connectable to a source of liquid for washing the material collected in the container, wherein there are further provided a source of liquid for treating the material collected in the collection container and valves for selectively coupling the two sources to the collection container.

The sources of fluid may be in the form of liquid containers connected to the collection container by means of a regulating and locking device. The collection container may consist of a cover provided with connecting elements, and a replaceable vessel provided with closing means usable after the vessel has been detached from the cover. At least the vessel may be disposable after use, being of e.g. plastics.

The collection container may be at least partly of transparent material and is advantageously provided with a bag-like filter insert suitably connected to the cover.

According to another aspect of the invention there is provided a method of washing and treating material removed from a cavity by suction, using the above apparatus.

By means of apparatus or a method in accordance with the invention, there is possible not only the continuous washing and filtering of material extracted from a body cavity by suction, so that the parts necessary for further examination can be separated in the suction apparatus itself without being damaged, and can be visually evaluated, but also treatment with a suitable liquid so that they can be prepared for further examination.

By providing for treatment with various liquids, the apparatus is suitable for preparation, fixing, disinfection, dying of examination material, or for preparation of cytochemical, histochemical, ultramicroscopic, or autoradiographic examinations etc. A further advantage is that by enabling rapid fixing if required autolysis of cell organelles is preventible.

Referring to the apparatus disclosed in U.S. Patent No. 3,721,244, a drawback — besides those already mentioned — is that the catheter is not replaceable at all. A further disadvantage is that for instance in case of interruption of pregnancy, in the interest of easy manipulation with the instrument the cervix should previously be dilated in relation to the diameter of the catheter.

In general, little consideration is given to the necessity of careful treatment of the cervix; replacement of a catheter during operation is not possible, or is difficult to achieve; and simultaneous — for instance cytological — sample taking is not possible in the course of the operation.

Highly significant among known suction catheters are those which are used for intrauterine interventions in gynaecology. Medical practice has special requirements concerning gynaecological suction catheters. The form of the end of the suction catheter should enable one to pass a catheter of necessary thickness through the cervix after minimal dilation and without any injury thereof. Injury of the cervix is very frequent, causing premature births, subsequent sterility and cancerous diseases. At the same time, for removal of more persistently sticking materials it is advisable to use rigid edge-like forms in such an arrangement by which the removal of endometrium persistently adhering to the rear wall of the uterus, can also be ensured. At the same time it would be essential, that the suction catheter should not cause injury, e.g. perforation of the uterus. In this respect the above described solutions are not entirely satisfactory.

Thus it is apparent that known rigid suction catheters leave many problems unsolved. Although the possibility of edge development is provided with instruments made from metal, the rigidity of the catheter frequently leads to injury of the uterus. For this reason flexible suction catheters, such as for instance the flexible catheter disclosed in US Patent No. 3,506,010 have become widely popular. These catheters considerably reduce the risk of injury, but their flexible material does not allow the rigid edge development, and thus they are not suitable for sample taking, or for the removal of persistently sticking materials. In U.S. Patent 3,774,612 there is disclosed a flexible catheter having a rigid end piece, but the tip of this is deliberately thinned to make it flexible and soft. Thus this arrangement does not solve the problem either.

Thus in an embodiment of the present invention there is provided a catheter which permits the removal of persistently sticking material particles, similarly to the method carried out with a curette scoop, and at the same time makes the excessive dilation of the cavity entrance avoidable and reduces the risk of injury. Thus there is used a flexible catheter in which the or each suction opening is surrounded at least in part by rigid material providing a hard edge.

In flexible plastics suction catheters the material surrounding the suction opening can be made rigid by known physical or chemical processes. Surrounding the suction opening with rigid material can also be provided by making the end of the suction catheter from metal. There are also possible arrangements in which only the parts surrounding the suction opening are formed as as an insert made from metal, or other rigid material.

In case of a suction catheter with a closed distal end, the end part, i.e. the end to be entered into the cavity, is preferably rounded asymmetrically.

In apparatus in accordance with the invention the catheter is preferably provided with a guide tube, its distal end positionable in the entrance of the cavity, so as to facilitate the penetration of the suction catheter into, and its controlled movement within, the cavity. The proximal end of the guide tube is preferably formed in such a way, that its moving and fixing are simple and safe.

The guide tube may consist of several parts fixed to each other, and furthermore it may be releasably fixed to the suction catheter.

The outer part of the guide tube may be provided with a handle and with position marks referring to the position in relation to the cavity entrance.

In apparatus in accordance with the invention the catheter is preferably not connected directly to the collection container, but via a suction tube. The use of a flexible suction tube is very advantageous in respect of free movement and feeling with the catheter.

The catheter side of the suction tube is preferably provided with a known connecting part, to which the catheter is simply connectable and thus easily replaceable.

The development of the catheter end as described above ensures that in the course of its introduction into the cavity, the risk of injury to the cavity entrance is minimal. The guide tube for introducing the catheter into the cavity also reduces the risk of damaging the cavity entrance. By its use, excessive dilation of the cervix is avoidable. Without use of the guide tube the cervix has to be considerably dilated, partly to provide easier manipulation and partly because of spontaneous contraction during operation. A further advantage is that by using the guide tube, material can be obtained from the cavity of the uterus without the injury of the hymen.

In this way the flexible catheter described above considerably reduces the injury of the cavity because of its flexibility, and at the same time with the aid of the suction openings provided with hard edges, the contents of the cavity including persistently sticking materials are easily removable.

In many cases, e.g. in gynaecological practice, on removal of the contents of the uterus, it is an important aspect, that no removable material should remain in the cavity. This requirement is met by the flexible catheter described above by simultaneously ensuring the removal of material with the suction holes provided with cutting edges, and its evacuation by the aid of the suction effect.

The advantage of an asymmetrically rounded catheter end is significant in its gynaecological application.

With this development, in the course of passing the catheter through the cervix, by turning the suction catheter while it is being pushed in, the risk of injuring the inner orifice of the uterus is considerably reduced.

The reason for this is found in the fact, that the inner orifice of the uterus is situated asymmetrically in relation to the line of the cervix, and thus with the asymmetrically rounded end of the suction catheter, the orifice of the inner uterus can be found when it is pushed in, the lip of the inner orifice is not in frontal contact with the catheter-end.

The asymmetrically rounded cather-end provides advantages in the case of other cavity entrances also, since by turning the cather, its end moves concentrically, thereby considerably facilitating the progress in the cavity entrance.

Some embodiments of the invention will now be described by way of example and with reference to the accompanying drawings, in which:—

Figure 1 is a schematic diagram of apparatus in accordance with the invention;

Figure 2 is a more detailed, schematic illustration of the apparatus shown in Fig. 1;

Figure 3a is a section of a catheter in accordance with the invention;

Figure 3b is a plan view of the catheter of Fig. 3a;

Figure 4a is a longitudinal section through another embodiment of a catheter in accordance with the invention;

Figure 4b is a transverse section through the catheter of Fig. 4a;

Figure 4c is a plan view of the catheter of Figs. 4a and 4b;

Figure 5a is a longitudinal section through a further embodiment of a catheter in accordance with the invention;

Figure 5b is a plan view of the catheter of Fig. 5a;

Figure 6 is a plan view of another catheter in accordance with the invention, partially in section;

Figure 7 shows a section through a guide tube for use with apparatus in accordance with the invention whilst being in use;

Figure 8a is a longitudinal section through another guide tube and catheter in accordance with the invention whilst being in use; and Figures 8b and 8c are two transverse sections through the arrangement of Fig. 8a.

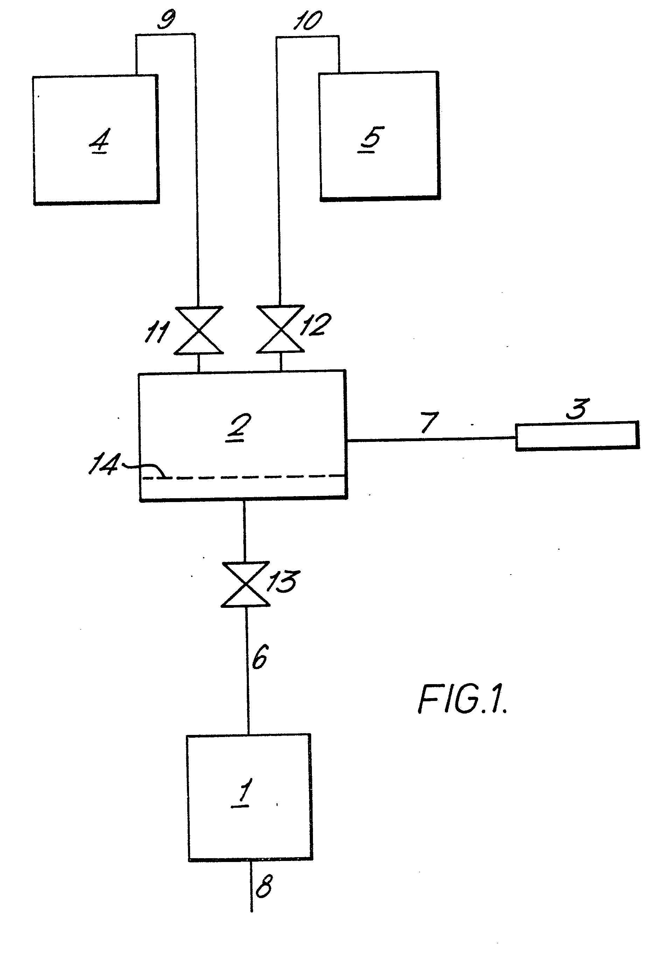

Referring to Figure 1, an ejector jet pump 1 with tubing 6 is connected to a material collection container 2. This is connected with a flexible catheter 3 by means of a suction tube 7. Tubing 8 is used for discharge of waste water from pump 1.

A washing liquid container 4 is connected to the collection container 2 by means of tubing 9, and a fixing liquid container 5 by means of tubing 10. The lengths of tubing 9, 10 and 6 are provided with valves 11, 12 and 13 respectively.

Operation of the apparatus is as follows—Ejector jet pump 1, with valve 13 at the open position, brings about a vacuum in the collection container 2. As a result of the vacuum, a suction effect develops at the suction openings of catheter 3, and depending on the positions of the valves 11 and 12, washing liquid and/or fixing liquid flows from containers 4 and 5 respectively into the collection container 2. The arrangement of containers 4 and 5 is such that their maximum liquid level is lower than the level of the collection container 2, and thus the flow of liquid will occur only in case of vacuum.

By introducing a catheter 3 into the uterus, the endometrium is removable, e.g. in the course of interruption of pregnancy. The sucked material passes through suction tube 7 into the collection container 2, where the washing liquid washes out the blood, while the tissue particles get caught on a built-in replaceable filter 14, and are thus withheld. The blood-laden washing water passes continuously through tubing 6, ejector jet pump 1 and tubing 8 into a sewage canal. The quantity and time of applying the washing liquid and fixing liquid may be chosen at discretion. After completion of the operation, the tissue parts, e.g. necessary for histological examination, separated from the blood, washed and fixed, are removable from the collection container 2.

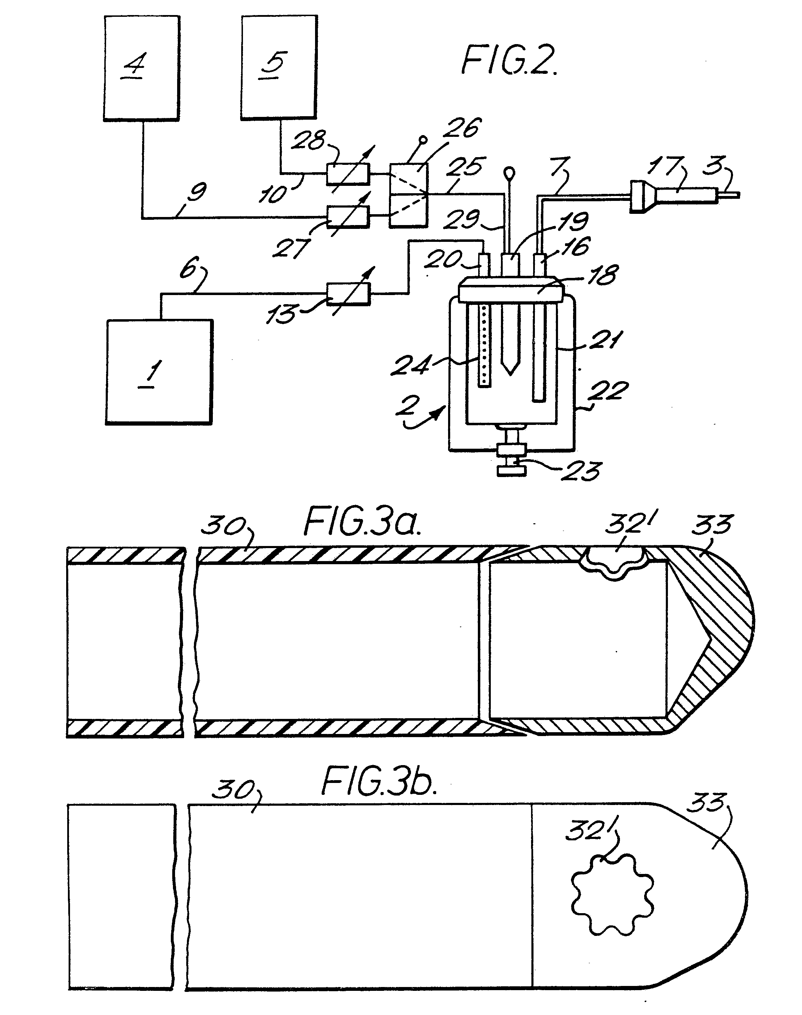

Figure 2 shows a possible structural design of the collection container 2 with the connected units. Catheter 3 is connected through suction tube 7 to stub 16 of the collection container 2. The catheter can be passed through the hole of a guide tube 17 and preferably fixed in it. A cover 18 of the collection container 2 is provided with a suspension element 29 for holding up the container. Connecting tube stubs 16, 19 and 20 are provided on cover 18. A transparent vessel 21 of the container 2, and cover 18, are locked airtight with a clamping element 23 of a fixing device 22. A bag-like filter insert 24 is provided on an extension of tube stub 20, reaching into the interior of vessel 21. Tubing 25 is connected to tube stub 19, leading to the outlet of a selector valve 26. Tubing 9, provided with a choke valve 27, is connected to one of the inlets of selector valve 26, the other end of the tubing 9 being connected to the washing liquid container 4. Tubing 10, provided with a choke valve 28, leading from fixing liquid container 5, is connected to the other inlet of selector valve 26. Tubing 6 provided with a valve 13 is connected to the tube stub 20 and leads to the ejector jet pump 1.

Ejector jet pump 1 brings about vacuum in the collection container 2, through tubing 6 at open position of valve 13. As a result of the vacuum, at one of the open positions of the selector valve 26 a quantity of the washing liquid — set by choke valve 27 — flows from the washing liquid container 4 through tubing 25 into container 2, and at the other open position a quantity of the fixing liquid — set by choke valve 28 — flows from the fixing liquid container 5 into the container 2. At the same time, at suction openings in the distal end of catheter 3 a suction effect develops, the magnitude depending on the positions of valves 13, 27, 28 and 26. Removal of the contents of a cavity takes place with the help of this suction effect in the course of operation with the apparatus as described by way of an example as follows.

The distal end of the guide tube 17 is introduced into the cervix. This is followed by passing catheter 3 through the hole of guide tube 17 and by removal of the contents of the uterus. The materials sucked out of the cavity pass through tube stub 16 into the washing-treating container 2, from where the liquid parts e.g. blood as a result of the continuous washing depart through tube stub 20, whilst the other parts e.g. tissues necessary for further examination, remain in vessel 21. Thus introduction of the washing liquid takes place simultaneously with removal of the contents of the uterus, while application of the fixing liquid may take place at the end of the removal, immediately after washing. The washed and fixed material may be stored in vessel 21 after releasing the clamping element 23. Vessel 21 can be closed with a closure element, suitably with a stopper.

The suction openings at the end of the suction catheter to be introduced into the cavity are provided with a knife-like edge. This can be attained in several ways. According to a preferred design the catheter is made from polyethylene tube and its end to be introduced into the cavity is hardened by a chemical process, e.g. by soaking in benzene, i.e. by dissolving the softeners. The sharp-edged suction opening is provided by the hardened material part.

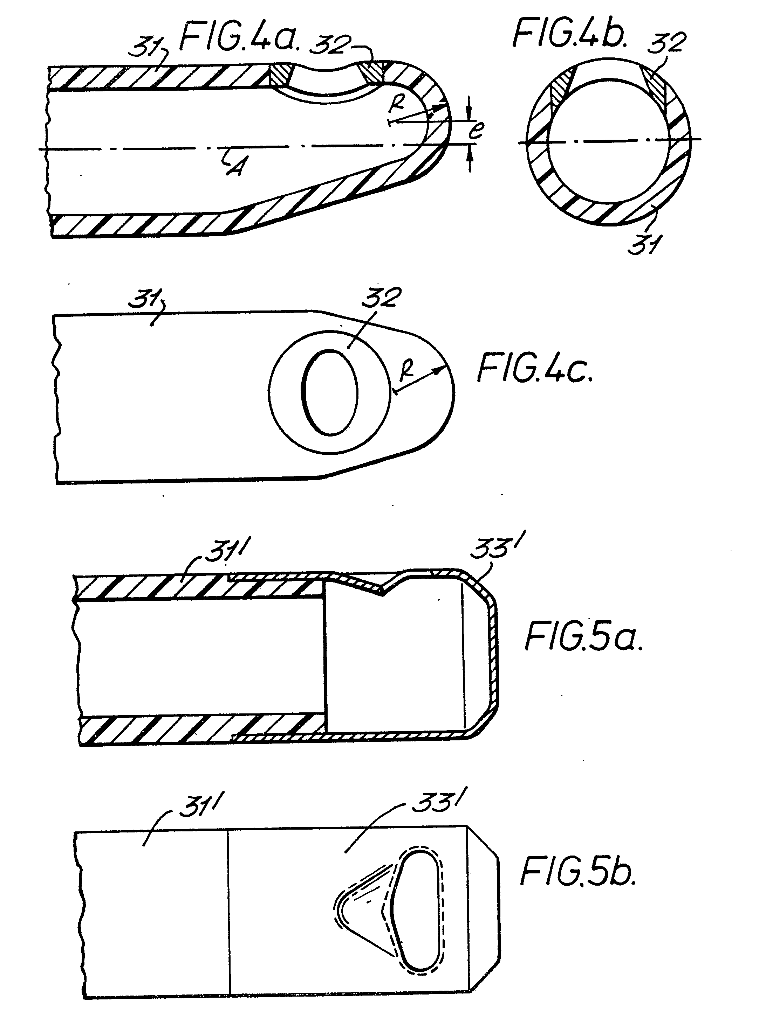

In one preferred embodiment of a structural design of catheter in accordance with the invention as shown in Figures 4a, b and c, a metal insert 32 is placed into a lateral suction opening of a flexible catheter tube 31, at the end of which a sharp orifice edge is thus developed, reaching to the periphery of the tube 31.

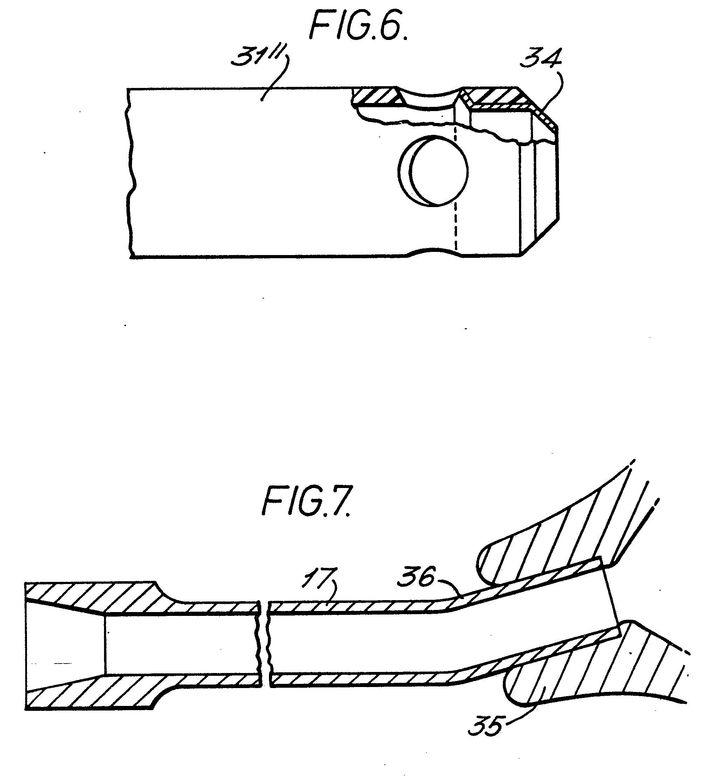

In a further embodiment shown in Figure 3a and 3b, the end of a flexible catheter tube 30 to be introduced into the cavity, has a tip 33, say 1 to 3 cm long, made from metal, and the lateral suction opening 32' is provided in this tip. A similar construction is shown in Fig. 5a and 5b, with a flexible catheter tube 31' being provided with a metal tip 33'.

Figure 6 shows a further design of a catheter in accordance with the invention. Here a metal brush 34 is fixed in the open end of a flexible catheter tube 31".

In this arrangement the metal bush 34, and thus the catheter too, is open in axial direction. Lateral openings are also provided.

According to a preferred embodiment of the invention, the suction catheter is closed in axial direction and is asymmetrically rounded. The provision of asymmetrical rounding means that centreline of the catheter tube and that of the rounding do not coincide. The enveloping surface of the rounding may be a surface of rotation, e.g. spherical surface. One form of the asymmetrical rounding can be seen in Figure 4a. In this arrangement the catheter tube ends in a sphere of radius R, arranged with eccentricity e with respect to the axis A of tube 31. Another example of asymmetrical rounding is seen in Figures 3a and 3b. In this arrangement the metal end 33 is rounded asymmetrically.

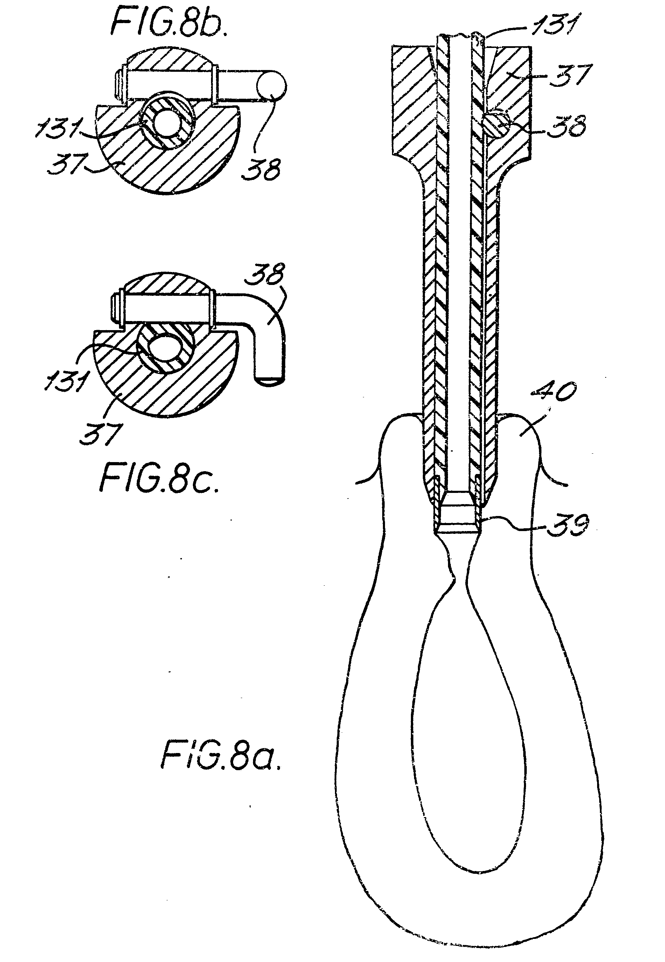

The catheter is preferably also provided with a guide tube. The guide tube 17 is used for introducing the catheter 3 into the cavity. The shape and size of the aperture extending the length of the guide tube 17 is such as to suit the shape and size of the suction catheter, so that the suction catheter can be easily passed through the guide tube. The external form at one end of the guide tube suits the shape and size of the cavity entrance, while the axial passage at the other end is preferably tapered, and its external shape may be handle-like. The guide tube can be seen clearly in Figure 7. One end 36 of the guide tube 17 fits into the cavity entrance 35. The catheter can be led easily into the cavity through the passage in the guide tube 17 in such a way that movement of the suction catheter is not prevented by the spontaneous contraction of the cavity entrance, without the risk of injury. Use of the guide tube is very advantageous in gynaecological practice. As can be seen, the proximal end of the guide tube passage is tapered, to assist feeding of the catheter.

The guide tube is introduced preferably only up the inner orifice of the uterus, and may be straight or slightly bent in respect of its centreline. Figure 7 shows a bent guide tube.

Figures 8a, b and c show another embodiment of a catheter 131 and a guide tube 37. A lock pin 38 is provided in a thickened part of guide tube 37. In one position of the lock pin as shown in Fig. 8b the catheter 131 can be freely moved in the guide tube. By turning the lock pin 38 to the position shown in Fig. 8c the catheter tube 131 can be fixed in guide tube 37. The catheter tube 131 is provided with metal tip 39 at its end to be led into the cavity.

This catheter development is especially suitable in gynaecology for taking samples from the cervical canal. Figure 8 shows such an application. The catheter, fixed with lock pin 38 in guide tube 37, in the course of its introduction into cervix 40 separates valuable examination material from the endoceruix. By turning the lock pin 38, catheter tube 131 is releasable and the operation, with or without a catheter change, can be continued with the same apparatus without interruption. In a preferable structural design of the guide tube, the visible part of the outer mantle outside the cavity entrance is provided with marks, e.g. notches with numerical symbols — in order to facilitate estimation of the position of the distal end of the guide tube and the inner orifice of the uterus in relation to each other.

## Claims

1. Apparatus for use in the removal of material from body cavities by suction, comprising a catheter (3), a suction unit (1), and a collection container (2) disposed between the catheter and the suction unit, said container being provided with a filter (14) in the flow path between the catheter and suction unit and being connectable to a source (4) of liquid for washing the material collected in the container, characterised by a source (5) of liquid for treating the material collected in the collection container (2) and valves (11, 12) for selectively coupling the two sources (4, 5) to the collection container.

2. Apparatus as claimed in claim 1, characterised in that the collection container (2) comprises a cover (18) and a replaceable vessel (21).

3. Apparatus as claimed in claim 2 characterised in that the filter is in the form of a bag (24) and is connectable to the cover (18).

4. Apparatus as claimed in claim 1, 2 or 3 characterised in that at least part (21) of the collection container (2) is transparent.

5. Apparatus as claimed in any preceding claim characterised in that the catheter (3) is connected to the collection container (2) by means of a flexible suction tube (7).

6. Apparatus as claimed in any preceding claim characterised in that the catheter (3) is provided with a guide tube (17) the distal end of which is positionable in a body cavity and which guides penetration of the catheter into the cavity.

7. Apparatus as claimed in any preceding claim characterised in that the catheter (30; 31) is flexible and has a suction opening (32'; 32) surrounded at least in part by rigid material providing a hard edge.

8. Apparatus as claimed in claim 7 characterised in that the distal end (33) of the catheter (30) is made of rigid material in which the suction opening (32) is provided.

9. Apparatus as claimed in claim 7, characterised in that an insert (32) of rigid material surrounds the suction opening in the catheter (31).

10. Apparatus as claimed in any preceding claim characterised in that the catheter (30) has a closed end and a lateral opening (32'), the closed end being asymmetrically rounded.

11. A method of washing and treating material removed from a body cavity by suction using a catheter (3), a suction unit (1), and a collection container disposed between the catheter and the suction unit, said container being provided with a filter (14) in the flow path between the catheter and suction unit and being connectable to a source (34) of liquid which is used to wash the material collected in the container, characterised in that after washing of the collected material the container (2) is connected to a source (5) of liquid which is used to treat the collected material, selective coupling of the container to the two sources (4, 5) being performed by means of valves (11, 12).

12. A method as claimed in claim 11, characterised in that the treating liquid is a fixing liquid for preserving the structure of the removed material.

## Revendications

1. Appareil destiné à être utilisé pour l'enlèvement de matière hors de cavités du corps par aspiration, comprenant un cathéter (3), un appareil aspirateur (1), et un récipient collecteur (2) disposé entre le cathéter et l'appareil aspirateur, ledit récipient étant muni d'un filtre (14) dans le trajet d'écoulement entre le cathéter et l'appareil aspirateur et pouvant être raccordé à une source (4) de liquide pour laver la matière recueillie dans le récipient, caractérisé par une source (5) de liquide pour traiter la matière recueillie dans le récipient collecteur (2) et des robinets (11, 12) pour raccorder sélectivement les deux sources (4, 5) au récipient collecteur.

2. Appareil tel que revendiqué dans la revendication 1, caractérisé en ce que le récipient collecteur (2) comprend un couvercle (18) et un vase remplaçable (21).

3. Appareil tel que revendiqué dans la revendication 2, caractérisé en ce que le filtre se présente sous la forme d'un sac (24) et peut être assemblé au couvercle (18).

4. Appareil tel que revendiqué dans les revendications 1, 2 ou 3, caractérisé en ce qu'au moins une partie (21) du récipient collecteur (2) est transparente.

5. Appareil tel que revendiqué dans l'une quelconque des revendications précédentes, caractérisé en ce que le cathéter (3) est raccordé au récipient collecteur (2) au moyen d'un tube d'aspiration flexible (7).

6. Appareil tel que revendiqué dans l'une quelconque des revendications précédentes, caractérisé en ce que le cathéter (3) est muni d'un tube de guidage (17) dont l'extrémité distale peut être positionnée dans la cavité du corps et qui guide la pénétration du cathéter dans la cavité.

7. Appareil tel que revendiqué dans l'une quelconque des revendications précédentes, caractérisé en ce que le cathéter (30, 31) est flexible et comporte une ouverture d'aspiration (32', 32) entourée au moins en partie par une matière rigide qui forme un bord dur.

8. Appareil tel que revendiqué dans la revendication 7, caractérisé en ce que l'extrémité distale (33) du cathéter (30) est fabriquée en une matière rigide dans laquelle l'ouverture d'aspiration (32) est formée.

9. Appareil tel que revendiqué dans la revendication 7, caractérisé en ce qu'une pièce rapportée (32) en matière rigide entoure l'ouverture d'aspiration formée dans le cathéter (31).

10. Appareil tel que revendiqué dans l'une quelconque des revendications précédentes, caractérisé en ce que le cathéter (30) a une extrémité fermée et une ouverture latérale (32'), l'extrémité fermée étant arrondie asymétriquement.

11. Un procédé de lavage et traitement d'une matière enlevée d'une cavité du corps par aspiration en utilisant un cathéter (3), un appareil aspirateur (1), et un recipient collecteur disposé entre le cathéter et l'appareil aspirateur, ce récipient étant muni d'un filtre (14) dans le trajet d'écoulement entre le cathéter et l'appareil aspirateur et pouvant être raccordé à une source (34) de liquide qui est utilisée pour laver la matière recueillie dans le récipient, caractérisé en ce qu'après lavage de la matière recueillie, le récipient (2) est raccordé à une source (5) de liquide qui est utilisé pour traiter la matière recueillie, le raccordement sélectif du récipient aux deux sources (4, 5) étant effectué au moyen de robinets (11, 12).

12. Un procédé tel que revendiqué dans la revendication 11, caractérisé en ce que le liquide de traitement est un liquide de fixation pour conserver la structure de la matière enlevée.

## Patentansprüche

1. Vorrichtung zur Verwendung bei der Entfernung von Stoffen aus Körperhöhlungen durch Absaugen, mit einem Katheter (3), einer Absaugeinheit (1) und einem Sammelbehälter (2), der sich zwischen dem Katheter und der Absaugeinheit befindet, wobei der Behälter einen Filter (14) im Fliesspfad zwischen dem Katheter und der Absaugeinheit enthält und mit einer Quelle (4) einer Flüssigkeit zum Waschen des in dem Behälter gesammelten Materials verbindbar ist, gekennzeichnet durch eine Quelle (5) einer Flüssigkeit zum Behandeln des in dem Sammelbehälter (2) gesammelten Materials und Ventilen (11, 12) zum selektiven Verbinden der zwei Quellen (4, 5) an den Sammelbehälter.

2. Vorrichtung gemäss Anspruch 1, dadurch gekennzeichnet, dass der Sammelbehälter (2) eine Abdeckung (18) und ein ersetzbares Gefäss (21) enthält.

3. Vorrichtung gemäss Anspruch 2, dadurch gekennzeichnet, dass das Filter in Form eines Beutels (24) vorliegt und mit der Abdeckung (18) verbindbar ist.

4. Vorrichtung gemäss Ansprüchen 1, 2 oder 3, dadurch gekennzeichnet, dass wenigstens ein Teil (21) des Sammelbehälters (2) transparent ist.

5. Vorrichtung gemäss einem der vorhergehenden Ansprüche, dadurch gekennzeichnet, dass der Katheter (3) mit dem Sammelbehälter (2) durch ein flexibles Absaugrohr (7) verbunden ist.

6. Vorrichtung gemäss einem der vorhergehenden Ansprüche, dadurch gekennzeichnet, dass der Katheter (3) mit einem Führungsrohr (17) verbunden ist, dessen entferntes Ende in eine Körperhöhlung eingebracht werden kann und als Führung für das Einbringen des Katheters in die Höhlung dient.

7. Vorrichtung gemäss einem der vorhergehenden Ansprüche, dadurch gekennzeichnet, dass der Katheter, (30, 31) flexibel ist und eine Absaugöffnung (32', 32) aufweist, die wenigstens zum Teil von einem harten Material mit harten Kanten umgeben ist.

8. Vorrichtung gemäss Anspruch 7, dadurch gekennzeichnet, dass das entfernte Ende (33) des Katheters (30) aus einem harten Material besteht, in welchem die Absaugöffnung (32) vorgesehen ist.

9. Vorrichtung gemäss Anspruch 7, dadurch gekennzeichnet, dass ein Einsatz (32) aus hartem Material die Absaugöffnung des Katheters (31) umgibt.

10. Vorrichtung gemäss einem der vorhergehenden Ansprüche, dadurch gekennzeichnet, dass der Katheter (30) ein geschlossenes Ende und eine laterale Öffnung (32') hat, wobei das geschlossene Ende asymmetrisch gerundet ist.

11. Verfahren zum Waschen und Behandeln von Materialien, die aus einer Körperhöhlung durch Absaugen entfernt wurden unter Verwendung eines Katheters (3), einer Absaugeinheit (1) und eines Sammelbehälters, der sich zwischen dem Katheter und der Absaugeinheit befindet, wobei der Behälter mit einem Filter (14) in dem Fliesspfad zwischen dem Katheter und der Absaugeinheit ausgerüstet ist und verbindbar an eine Quelle (34) der Flüssigkeit, die zum Waschen des in dem Behälter verwendeten Materials verwendet wird, ist, dadurch gekennzeichnet, dass nach dem Waschen des gesammelten Materials der Behälter (2) mit einer Quelle (5) einer Flüssigkeit verbunden wird, die zum Behandeln des gesammelten Materials verwendet wird, wobei eine selektive Verbindung des Behälters an die beiden Quellen (4, 5) mittels der Ventile (11, 12) durchgeführt wird.

12. Verfahren gemäss Anspruch 11, dadurch gekennzeichnet, dass die Behandlungsflüssigkeit eine Fixierflüssigkeit zum Bewahren der Struktur des entfernten Materials ist.

0 001 718

FIG.1.

FIG.2.

FIG.3a.

FIG.3b.

FIG.4a.

31  32

R

e

A

FIG.4b.

32

31

FIG.4c.

31

32

R

FIG.5a.

31'  33'

FIG.5b.

31'  33'

## FIG.6.

31'''

34

## FIG.7.

17

36

35

FIG.8b.

FIG.8c.

FIG.8a.